(19) Europäisches Patentamt
European Patent Office
Office européen des brevets

(11) **EP 3 982 827 B1**

(12) **EUROPEAN PATENT SPECIFICATION**

(45) Date of publication and mention
of the grant of the patent:
**21.06.2023  Bulletin 2023/25**

(21) Application number: **20726979.6**

(22) Date of filing: **12.05.2020**

(51) International Patent Classification (IPC):
*A61B 5/08* *(2006.01)*    *A61B 5/087* *(2006.01)*
*A61B 5/00* *(2006.01)*    *A61B 5/103* *(2006.01)*
*G01N 21/78* *(2006.01)*    *G01N 33/497* *(2006.01)*

(52) Cooperative Patent Classification (CPC):
**A61B 5/082; A61B 5/087; A61B 5/0873;**
**A61B 5/1032; A61B 5/1034; A61B 5/7267;**
**G01N 21/783; G01N 33/497;** A61B 2560/0223

(86) International application number:
**PCT/SE2020/050483**

(87) International publication number:
**WO 2020/251440 (17.12.2020 Gazette 2020/51)**

(54) **CAPNOSCOPE ASSEMBLY, AND A METHOD IN RELATION TO THE CAPNOSCOPE ASSEMBLY**

KAPNOSKOPANORDNUNG UND VERFAHREN ZUR HERSTELLUNG EINES KAPNOSKOPS

ENSEMBLE CAPNOSCOPE ET PROCÉDÉ ASSOCIÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.06.2019  SE 1950696**

(43) Date of publication of application:
**20.04.2022  Bulletin 2022/16**

(73) Proprietor: **Sensebreath AB
148 96 Sorunda (SE)**

(72) Inventor: **GEDEON, Andras
114 31 Stockholm (SE)**

(74) Representative: **Bjerkén Hynell KB
Tulegatan 53
113 53 Stockholm (SE)**

(56) References cited:
**US-A1- 2016 125 600    US-A1- 2016 331 271
US-B2- 10 080 510    US-B2- 10 175 254**

EP 3 982 827 B1

**Description**

Technical field

The present disclosure relates to a capnoscope assembly, and a method in relation to the assembly, intended to measure CO2 concentrations in a gas, and in particular to a calibration procedure in relation to the capnoscope assembly.

Background

The technique of using miniature cameras for viewing difficult-to-reach places is well established. For instance borescopes are used in construction, endoscopes are used to view the cavities inside the human body and otoscopes are used to view the ear drums. A large number of devices are available from many manufacturers.

Lately the performance of these devices have dramatically improved and their size and cost have been much reduced. They now offer high resolution, built in thermal control, adjustable light emitting diode (LED) illumination and allow to be submersed. They are rugged, communicate using built in WiFi with any of the common type of smartphone or computer protocols.

Although they are designed to produce images/videos of the surroundings they can also be adapted to show the colour changes of an indicator surface. If an indicator is attached to the device that changes colour in response to CO2 gas, then this assembly is called a capnoscope. The use of such a device is denoted capnoscopy. Optical readings of CO2 indicators are well established technology.

The following patent documents comprise a comprehensive list of relevant disclosures of related technology.

US-10175254 relates to methods and systems for quantitative colorimetric capnometry. The disclosed system includes a gas conduit, a colorimetric indicator adapted to exhibit a colour change in response to exposure to carbon dioxide gas. A measurement signal of the colour change is provided and a concentration of carbon dioxide based on the measurement signal may be computed.

US-10080510 relates to a breath analysing and training assembly for detecting CO2 concentration in breathing gas of a user, including a selective colorimetric CO2 detector having a detector surface which rapidly and reversibly changes colour with CO2 concentration. A processing element measures CO2 concentration changes in the breathing gas by identifying colour changes of images of a detector surface captured by an image capturing element.

US-2016/0331271 relates to a manual resuscitator and capnography assembly that is configured to measure carbon dioxide concentration in breathing gas of person being ventilated.

Accurate colorimetric CO2 determination requires that the device can be set to zero when no CO2 is present and that the device is calibrated such that is shows correct values at a suitably chosen concentration of CO2.

While the first condition is easy to achieve the second requires the use of a calibration gas and thus the availability and handling of a gas mixture with well-known CO2 concentration. This last requirement is a significant drawback when using the capnoscope in real life situations, for instance when monitoring the CO2 level in the expired breath of a patient in an ambulance or during anaesthesia in an operating room.

One object of the present invention is aimed at making calibration of a capnoscope without calibration gas possible. A more general object of the present invention is to achieve an improved capnoscope, wherein the improvement lies in providing an easy to use, and reliable capnoscope capable of performing accurate CO2 measurements.

Summary

The above-mentioned objects are achieved by the present invention according to the independent claims.

Preferred embodiments are set forth in the dependent claims.

The present invention relates to a first aspect where a capnoscope assembly is provided for measuring carbon dioxide (CO2) concentration.

The assembly comprises:

- a detection unit (2) comprising an illumination arrangement (4) and a camera (6) configured to capture colours of an indicator surface illuminated by light emitted by said illumination arrangement, wherein said illumination arrangement and camera are closely mounted together,
- an indicator holder (8) configured to hold a colorimetric indicator (10) having an indicator surface in front of the detection unit (2) in a predetermined position in relation to said detection unit (2),
- a processing unit (12) configured to control light parameters, e.g. the colour and intensity, of the light emitted by the illumination arrangement (4) by generating control signals (14). The capnoscope assembly further comprises a calibration member (16), and that said indicator holder (8) is configured to receive and hold said calibration member (16) in front of the detection unit (2) and in said predetermined position in relation to the detection unit (2), the calibration member (16) comprises a calibration surface having a predetermined calibration colour corresponding to the colour of an ideal reference indicator when subjected to a predefined $CO_2$ concentration that is not zero, wherein, when said calibration member is arranged in front of the detection unit (2), the processing unit (12) is configured to adjust the light parameters such that the camera (6) detects the predetermined calibration colour, and to determine and store the control signal values required to achieve that the predetermined calibration colour was detected, as calibrated control signal values, wherein the processing unit (12) is configured to apply a calibration procedure that comprises:

- arranging a colorimetric indicator (10) to be used in said indicator holder (8),
- measuring the colour of the colorimetric indicator surface when illuminated by emitted light being generated by using said determined calibrated control signal values, when no $CO_2$ is present, and storing said measured colour, as a zero $CO_2$ colour value, and
- determining a $CO_2$ concentration relationship based upon said measured zero $CO_2$ colour value, wherein said processing unit (12) is configured to apply said $CO_2$ concentration relationship during $CO_2$ concentration measurements provided that the determined calibrated control signal values are applied when emitting light.

The present invention also relates to a second aspect where a method in relation to a capnoscope assembly is provided for measuring carbon dioxide ($CO_2$) concentration. The method comprises receiving and holding a calibration member (16), in said indicator holder (8), in front of the detection unit (2) and in said predetermined position in relation to the detection unit (2), the calibration member (16) comprises a calibration surface having a predetermined calibration colour corresponding to the colour of an ideal reference indicator when subjected to a predefined $CO_2$ concentration that is not zero, wherein, when said calibration member is arranged in front of the detection unit (2), the method comprises adjusting the light parameters such that the camera (6) detects the predetermined calibration colour, and determining and storing the control signal values required to achieve that the predetermined calibration colour was detected, as calibrated control signal values, and applying a calibration procedure that comprises:

- arranging a colorimetric indicator (10) to be used in said indicator holder (8),
- measuring the colour of the colorimetric indicator surface when illuminated by emitted light being generated by using said determined calibrated control signal values, when no $CO_2$ is present, and storing said measured colour, as a zero $CO_2$ colour value, and
- determining a $CO_2$ concentration relationship based upon said measured zero $CO_2$ colour value, and the method further comprises applying said $CO_2$ concentration relationship during $CO_2$ concentration measurements provided that the determined calibrated control signal values are applied when emitting light.

Thus, a miniature camera and illumination arrangement is employed to analyse the colour of an indicator that reacts reversibly to the presence of carbon dioxide ($CO_2$). This device, a capnoscope assembly, can therefore measure the concentration of $CO_2$. In order to allow simple use and accurate results, the capnoscope must be calibrated. The present invention makes it possible to achieve this without the use of calibration gases.

With a calibration member comprising a printed reference surface for standardizing the use of a capnoscope assembly and with corrections to the measured colour, which is calculated based on the measured difference between the colour at zero $CO_2$ concentration and the color of an ideal reference indicator at zero $CO_2$ concentration, the assembly can be calibrated without the need of calibration gases. All that must be done before use is to determine the colour when no $CO_2$ is present.

Brief description of the drawings

Figure 1 is a block diagram schematically illustrating the capnoscope assembly according to the present invention.
Figure 2 is a schematic illustration of an exploded view of the capnoscope assembly according to one embodiment of the present invention.
Figure 3 is a schematic illustration of a perspective view of the detection unit according to one embodiment of the present invention.
Figure 4 is a flow diagram showing the method according to the present invention.

Detailed description

The capnoscope assembly will now be described in detail with references to the appended figures. Throughout the figures the same, or similar, items have the same reference signs. Moreover, the items and the figures are not necessarily to scale, emphasis instead being placed upon illustrating the principles of the invention.

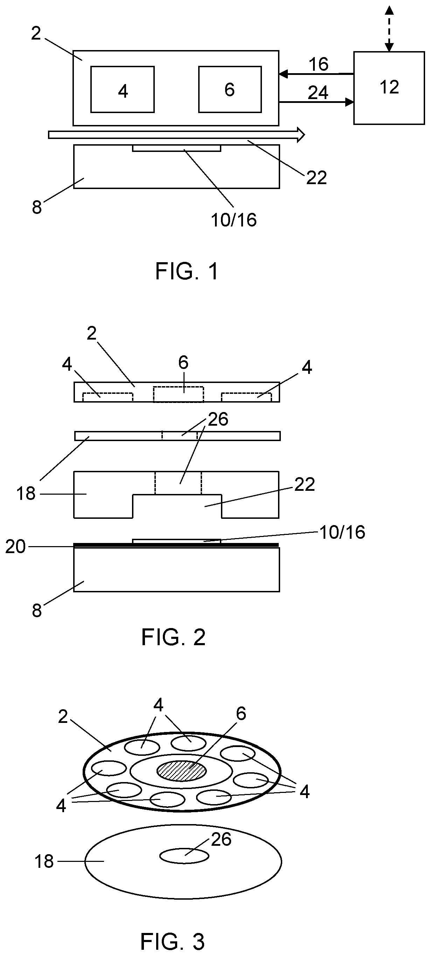

With references to figures 1-3 the capnoscope assembly for measuring carbon dioxide (CO2) concentration will now be described in detail. The capnoscope assembly comprises a detection unit 2 comprising an illumination arrangement 4 and a camera 6 configured to capture colours of an indicator surface illuminated by light emitted by the illumination arrangement. The illumination arrangement and camera are closely mounted together in a housing.

The assembly further comprises an indicator holder 8 configured to hold a colorimetric indicator 10 having an indicator surface in front of the detection unit 2 in a predetermined position in relation to the detection unit 2. The predetermined position is such that the indicator surface is positioned such that it is illuminated by light emitted by the illumination arrangement, and that the camera may capture colours of the indicator surface.

The assembly also comprises a processing unit 12 configured to control light parameters, e.g. the colour and intensity, of the light emitted by the illumination arrangement 4 by generating control signals 14 and applying the control signals to the illumination arrangement.

The processing unit is also configured to receive measurement signals 24 from the detection unit 2, and is provided with various processing and communication circuitry required to perform processing of received measurement signals, control of light parameters, and also to perform communication with external equipment(s). The communication is preferably wireless, e.g. WiFi, Bluetooth or any available communication protocol, but may also be performed via wire. The communication with an external equipment is indicated by a double arrow in figure 1.

The various parts of the detection unit is preferably energized by an energy member (not shown), e.g. a rechargeable battery, and the processing unit is then also provided with necessary circuitry for providing power to the other units, and also to monitor battery function and/or charging.

The camera may have various standard video resolutions (i.e. 640x480 or 1280x720) and focal lengths (typically 1.5 to 6 cm) and will allow a video with normally 10-30 frames per second. The illumination may be provided by a set of light emitting diodes (LED:s) (typically 6-8 LED:s) arranged around the lens of the camera - this is further discussed in relation to figure 3. They usually provide white light but can also use other colours. The illumination can be changed continuously by the processing unit, either automatically or in response of manual input.

The capnoscope assembly further comprises a calibration member 16, preferably having the same size as the colorimetric indicator 10, and that the indicator holder 8 is configured to receive and hold the calibration member 16 in front of the detection unit 2 and in the predetermined position in relation to the detection unit 2.

The calibration member 16 comprises a calibration surface having a predetermined calibration colour corresponding to the colour of an ideal reference colorimetric indicator when subjected to a predefined CO2 concentration that is not zero. When the calibration member is arranged in front of the detection unit 2, the processing unit 12 is configured to adjust the light parameters such that the camera 6 detects the predetermined calibration colour, and to determine and store the control signal values required to achieve that the predetermined calibration colour was detected, as calibrated control signal values.

The processing unit 12 is configured to apply a calibration procedure that comprises:

- arranging a colorimetric indicator 10 to be used in the indicator holder 8,
- measuring the colour of the colorimetric indicator surface when illuminated by emitted light being generated by using the determined calibrated control signal values, when no CO2 is present, and storing the measured colour, as a zero CO2 colour value, and
- determining a CO2 concentration relationship based upon the measured zero CO2 colour value, and in particular the deviation of this value from that of the ideal reference indicator.

Then, the processing unit 12 is configured to apply the CO2 concentration relationship during CO2 concentration measurements provided that the determined calibrated control signal values are applied when emitting light.

According to one embodiment, the illumination arrangement 4 comprises at least one light emitting diode (LED) arranged close to the camera 6, preferably symmetrically and/or circumferentially arranged around the camera. In figure 3 is illustrated an exploded view from below of an example where eight LED:s are arranged around the camera.

The assembly preferably also comprises an illumination modifying adaptor 18 configured to modify the emitted light, by providing diffused light and providing an aperture 26 to limit the field of view, in order to obtain images of sufficiently homogenous colour fields. Advantageously, the adaptor is needed for turning the spot type LED-illumination into a diffuse type of illumination and at the same time add an aperture to limit the field of view. Both of these conditions are desirable to obtain images of homogeneous colour fields.

In one embodiment the indicator holder 8 is configured to by releasably attachable to, and easily detachable from, the detection unit 2, preferably by a double adhesive member 20, e.g. a double adhesive tape or Velcro. The double adhesive member 20 can then also used when storing the colorimetric indicator prior to use by holding a protective aluminium foil in place. The adaptor 18 is provided with a gas passage 22 configured to allow a gas flow to be measured to reach the indicator surface. The gas flow may be kept very low, typically 1 ml/min. since the volume where the gas to be analysed passes can be minimal, typically only 0.005 ml (0.005 cc).

According to one embodiment the predefined CO2 concentration that is not zero is within the range of 4-7%, and preferably approximately 6%.

Preferably, the predetermined calibration colour is defined according to the RGB colour code system, and that only one of the RGB-parameters is used to define the calibration colour, preferably the R-parameter. Any of the other parameters of the RGB colour code system may naturally also be applied.

According to one embodiment the CO2 concentration relationship depends on R and the difference (delta) between the measured zero CO2 colour value and the zero CO2 colour value of the ideal reference colorimetric indicator. The CO2 colour concentration is calculated from the colour of the colorimetric indicator using the formula: $C(CO2) = A \times \ln (B/(B-(R-R0)))$, where $C(CO2)$ is the CO2 concentration, A and B are constants and R0 is the R value when no CO2 is present, provided that only the R-parameter in the RGB colour system is used.

Furthermore, the concentration relationship preferably has a linear form of the type R=R measured + delta $\times$ (K1 +K2 $\times$ R measured) where K1 and K2 are constants, and wherein the linear form for the R-value is applied when calculating the CO2 concentration using the formula in claim 6.

In the following a detailed discussion is included of one example of how to determine the CO2 concentration relationship.

A colorimetric indicator changes colour with the concentration of carbon dioxide (C).Thus, there is a relationship between the concentration of the gas and the colour of the indicator which can be expressed generally as:

$$C(CO2) = F(R,G,B)$$

F is a function of the R,G,B values that are an established way to define a colour and where each may have a value between 0 and 255.

The RGB colour model is an additive colour model in which red, green and blue light are added together in various ways to reproduce a broad array of colours. The name of the model comes from the initials of the three additive primary colours, red, green, and blue. The main purpose of the RGB colour model is for the sensing, representation and display of images in electronic systems, such as televisions and computers, though it has also been used in conventional photography. Before the electronic age, the RGB colour model already had a solid theory behind it, based in human perception of colours.

Also other colour models are naturally applicable herein, e.g. the CMYK colour model.

In practice it is sufficient to consider only one of these parameters, say the R-value, to characterize the colour relative to the concentration of CO2 and so:

$$C(CO2) = F(R)$$

For a given type of indicator the function F can be established by using a set of known concentration of CO2. The function may be for instance of the form

$$C(CO2) = A \times \ln (B/(B-(R-R0))) \qquad (1)$$

where A and B are constants and R0 is the R value when no CO2 is present, that is C=0.

In order for equation (1) to give 100% CO2 when R has its maximal value of 255 the B-value needs to be:

$$B=255-R0 + \text{epsilon}$$

where epsilon is typically ~ 0.001 and can therefore be neglected, for the clinical concentration range of 0-10 % CO2, resulting in

$$B=255-R0 \qquad (2)$$

The R - value for a given concentration of CO2 depends, apart from the properties of the indicator, also on the colour reading system including the illumination used.

Since only the properties of the indicator should determine the R-value, the other effects must be eliminated.

The properties of the colour reading instrumentation can be normalized at a certain chosen CO2 value, preferably in the upper part of the clinical range, for instance at C=6.0%.

This concentration is obtained from equation (1) if R0=38 and therefore B=217, (equation (2)) together with R6=150 and A= 8.26.

This set of values define the ideal reference indicator at 6.0% CO2 measured with an ideal reference instrument.

Then a test surface is printed, herein denoted calibration member 16, which matches the colour of the reference indicator at 6% concentration.

Next the printed test surface is arranged in another instrument, which does not necessarily show the expected R=150 but possibly a different value say R'.

Thereafter, the instrument is adjusted, for instance by changing the colour or the intensity of the illumination, so that it shows R'=150 for the printed reference surface. In this way the colour reading instrument is aligned with the ideal reference instrument.

The printed reference surface is then replaced with the colorimetric indicator to be used and the R-value at zero CO2 concentration, the R0 value, is determined.

To compensate for any difference between the indicator and the ideal reference indicator we must now compare R0 to the reference indicator at zero concentration, R0=38. The difference, Delta, will indicate the deviation between the two:

$$\text{Delta} = 38 - R0$$

This difference may be due to variations in the production process of the colorimetric indicators or a gradual change of indicator properties with time or both.

However, the deviation denoted by Delta at zero CO2 concentration is related in a known way, through a function U, to the deviations in R at other concentrations and this allows us to correct R over the whole range.

$$\text{Rcorr} = U(R, \text{Delta}), \text{ where U is a known function of R and Delta,} \qquad (3)$$

After this correction the indicator is aligned with the reference indicator.

For instance, for a specific type of indicator, the following applies:

$$\text{Rcorr} = R + \text{Delta} \times (1 + (R-R0)/(R6-R0)) \qquad (4)$$

This means that for these type of indicators the difference between an indicator and the ideal reference indicator is two times larger at 6% CO2 than at 0 %CO2.

In this way, using the Rcorr value, in the equation that applies to the ideal reference indicator described above the concentration of CO2 is obtained as:

$$C (CO2) = 8.26 \times \ln (217/(217- (Rcorr-38))) \qquad (5)$$

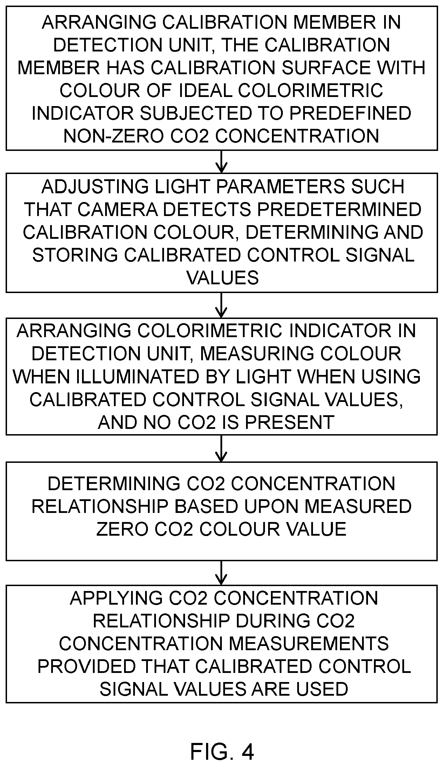

The present invention also relates to a method in relation to a capnoscope assembly for measuring carbon dioxide (CO2) concentration. The capnoscope assembly has been described in detail above, and it is here referred to that description. The method will now be described in detail with references to the flow diagram shown in figure 4.

Thus, the capnoscope assembly comprises:

- a detection unit 2 comprising an illumination arrangement 4 and a camera 6 configured to capture colours of an indicator surface illuminated by light emitted by said illumination arrangement, wherein said illumination arrangement and camera are closely mounted together,
- an indicator holder 8 configured to hold a colorimetric indicator 10 having an indicator surface in front of the detection unit 2 in a predetermined position in relation to said detection unit 2,
- a processing unit 12 configured to control light parameters, e.g. the colour and intensity, of the light emitted by the illumination arrangement 4 by generating control signals 14.

The method comprises receiving and holding a calibration member 16, in said indicator holder 8, in front of the detection unit 2 and in said predetermined position in relation to the detection unit 2. The calibration member 16 comprises a calibration surface having a predetermined calibration colour corresponding to the colour of an ideal reference indicator when subjected to a predefined CO2 concentration that is not zero. When the calibration member is arranged in front of the detection unit 2, the method comprises adjusting the light parameters such that the camera 6 detects the predetermined calibration colour, and determining and storing the control signal values required to achieve that the predetermined calibration colour was detected, as calibrated control signal values.

The method further comprises applying a calibration procedure that comprises:

- arranging a colorimetric indicator 10 to be used in said indicator holder 8,
- measuring the colour of the colorimetric indicator surface when illuminated by emitted light being generated by using said determined calibrated control signal values, when no CO2 is present, and storing said measured colour, as a zero CO2 colour value, and
- determining a CO2 concentration relationship based upon said measured zero CO2 colour value, and the method further comprises applying said CO2 concentration relationship during CO2 concentration measurements provided that the determined calibrated control signal values are applied when emitting light.

According to an embodiment of the method, the CO2 concentration relationship depends on R and the difference (delta) between the measured zero CO2 colour value and the zero CO2 colour value of the ideal reference indicator. The CO2 colour concentration is calculated from the colour of the colorimetric indicator using the formula: C (CO2) = A × ln (B/(B-(R-R0)), where C (CO2) is the CO2 concentration, A and B are constants and R0 is the R value when no CO2 is present, provided that only the R-parameter in the RGB colour system is used. The concentration relationship has advantageously a linear form of the type R=R measured + delta × (K1 +K2 × R measured) where K1 and K2 are constants, and wherein the linear form for the R-value is applied when calculating the CO2 concentration using the formula above.

The above calculations related to how the CO2 concentration relationship is determined is further discussed above in relation to the detailed discussion in relation to the example. The present invention is not limited to the above-described preferred embodiments.

The invention is defined by the appended claims.

## Claims

1. A capnoscope assembly for measuring carbon dioxide (CO2) concentration, comprising:

   - a detection unit (2) comprising an illumination arrangement (4) and a camera (6) configured to capture colours of an indicator surface illuminated by light emitted by said illumination arrangement, wherein said illumination arrangement and camera are closely mounted together,
   - an indicator holder (8) configured to hold a colorimetric indicator (10) having an indicator surface in front of the detection unit (2) in a predetermined position in relation to said detection unit (2),
   - a processing unit (12) configured to control light parameters, e.g. the colour and intensity, of the light emitted by the illumination arrangement (4) by generating control signals (14), **characterized in that** the capnoscope assembly further comprises a calibration member (16), and that said indicator holder (8) is configured to receive and hold said calibration member (16) in front of the detection unit (2) and in said predetermined position in relation to the detection unit (2), the calibration member (16) comprises a calibration surface having a predetermined calibration colour corresponding to the colour of an ideal reference colorimetric indicator when subjected to a predefined CO2 concentration that is not zero, wherein, when said calibration member is arranged in front of the detection unit (2), the processing unit (12) is configured to adjust the light parameters such that the camera (6) detects the predetermined calibration colour, and to determine and store the control signal values required to achieve that the predetermined calibration colour was detected, as calibrated control signal values, wherein the processing unit (12) is configured to apply a calibration procedure that comprises:

     - arranging a colorimetric indicator (10) to be used in said indicator holder (8),
     - measuring the colour of the colorimetric indicator surface when illuminated by emitted light being generated by using said determined calibrated control signal values, when no CO2 is present, and storing said measured colour, as a zero CO2 colour value, and
     - determining a CO2 concentration relationship based upon said measured zero CO2 colour value, wherein said processing unit (12) is configured to apply said CO2 concentration relationship during CO2 concentration measurements provided that the determined calibrated control signal values are applied when emitting light.

**2.** The capnoscope assembly according to claim 1, wherein said illumination arrangement (4) comprises light emitting diodes (LEDs) arranged close to said camera (6), preferably symmetrically and/or circumferentially arranged around said camera, and
wherein said assembly also comprises an illumination adjusting adaptor (18) configured to be controlled to adjust the emitted light, by providing diffused light and providing an aperture to limit the field of view, in order to obtain images of sufficiently homogenous colour fields.

**3.** The capnoscope assembly according to claim 1 or 2, wherein said indicator holder (8) is configured to by releasably attachable and easily detachable to said detection unit (2), preferably by a double adhesive member (20), and is provided with a gas passage (22) configured to allow a gas flow to be measured to reach said indicator surface.

**4.** The capnoscope assembly according to any of claims 1-3, wherein said predefined $CO_2$ concentration that is not zero is within the range of 4-7%, and preferably approximately 6%.

**5.** The capnoscope assembly according to any of claims 1-4, wherein said predetermined calibration colour is defined according to the RGB colour code system, and that only one of the RGB-parameters is used to define the calibration colour, preferably the R-parameter.

**6.** The capnoscope assembly according to any of claims 1-5, wherein said $CO_2$ concentration relationship depends on R and the difference (delta) between the said measured zero $CO_2$ colour value and the zero $CO_2$ colour value of the ideal reference colorimetric indicator, the $CO_2$ concentration is calculated from the colour of the colorimetric indicator using the formula: $C (CO_2) = A \times \ln (B/(B-(R-R0))$, where $C (CO_2)$ is the $CO_2$ concentration, A and B are constants and R0 is the R value when no $CO_2$ is present, provided that only the R-parameter in the RGB colour system is used.

**7.** The capnoscope assembly according to claim 6, wherein said concentration relationship has a linear form of the type R=R measured + delta × (K1 +K2 × R measured) where K1 and K2 are constants, and wherein said linear form for the R-value is applied when calculating the $CO_2$ concentration using the formula in claim 6.

**8.** A method in relation to a capnoscope assembly for measuring carbon dioxide ($CO_2$) concentration, the capnoscope assembly comprises:

- a detection unit (2) comprising an illumination arrangement (4) and a camera (6) configured to capture colours of an indicator surface illuminated by light emitted by said illumination arrangement, wherein said illumination arrangement and camera are closely mounted together,
- an indicator holder (8) configured to hold a colorimetric indicator (10) having an indicator surface in front of the detection unit (2) in a predetermined position in relation to said detection unit (2),
- a processing unit (12) configured to control light parameters, e.g. the colour and intensity, of the light emitted by the illumination arrangement (4) by generating control signals (14), **characterized in that** the method comprises receiving and holding a calibration member (16), in said indicator holder (8), in front of the detection unit (2) and in said predetermined position in relation to the detection unit (2), the calibration member (16) comprises a calibration surface having a predetermined calibration colour corresponding to the colour of an ideal reference colorimetric indicator when subjected to a predefined $CO_2$ concentration that is not zero, wherein, when said calibration member is arranged in front of the detection unit (2), the method comprises adjusting the light parameters such that the camera (6) detects the predetermined calibration colour, and determining and storing the control signal values required to achieve that the predetermined calibration colour was detected, as calibrated control signal values, and applying a calibration procedure that comprises:

- arranging a colorimetric indicator (10) to be used in said indicator holder (8),
- measuring the colour of the colorimetric indicator surface when illuminated by emitted light being generated by using said determined calibrated control signal values, when no $CO_2$ is present, and storing said measured colour, as a zero $CO_2$ colour value, and
- determining a $CO_2$ concentration relationship based upon said measured zero $CO_2$ colour value, and the method further comprises applying said $CO_2$ concentration relationship during $CO_2$ concentration measurements provided that the determined calibrated control signal values are applied when emitting light.

**9.** The method according to claim 8, wherein said $CO_2$ concentration relationship depends on R and the difference (delta) between the said measured zero $CO_2$ colour value and the zero CO2 colour value of the ideal reference colorimetric indicator, the CO2 colour concentration is calculated from the colour of the colorimetric indicator using the formula: $C(CO2) = A \times \ln(B/(B-(R-R0))$, where $C(CO2)$ is the CO2 concentration, A and B are constants and R0 is the R value when no CO2 is present, provided that only the R-parameter in the RGB colour system is used.

10. The method according to claim 9, wherein said concentration relationship has a linear form of the type R=R measured + delta $\times$ (K1 +K2 $\times$ R measured) where K1 and K2 are constants, and wherein said linear form for the R-value is applied when calculating the CO2 concentration using the formula in claim 9.

**Patentansprüche**

1. Kapnoskopanordnung zur Messung der Kohlendioxid(CO2)-Konzentration, umfassend:

- eine Detektionseinheit (2), umfassend eine Beleuchtungsanordnung (4) und eine Kamera (6), die ausgestaltet ist, Farben einer Indikatoroberfläche zu erfassen, die mittels Licht beleuchtet wird, das von der Beleuchtungsanordnung ausgestrahlt wird, wobei die Beleuchtungsanordnung und die Kamera nah beieinander montiert sind,
- eine Indikatorhalterung (8), die ausgestaltet ist, einen kolorimetrischen Indikator (10), der eine Indikatoroberfläche aufweist, vor der Detektionseinheit (2) in einer festgelegten Position in Bezug auf die Detektionseinheit (2) zu halten,
- eine Verarbeitungseinheit (12), die ausgestaltet ist, Lichtparameter, z. B. die Farbe und Intensität, des von der Beleuchtungsanordnung (4) ausgestrahlten Lichts zu steuern, indem sie Steuersignale (14) erzeugt, **dadurch gekennzeichnet, dass** die Kapnoskopanordnung ferner ein Kalibrierelement (16) umfasst, und dass die Indikatorhalterung (8) ausgestaltet ist, das Kalibrierelement (16) aufzunehmen und vor der Detektionseinheit (2) und in der festgelegten Position in Bezug auf die Detektionseinheit (2) zu halten, wobei das Kalibrierelement (16) eine Kalibrieroberfläche umfasst, die eine festgelegte Kalibrierungsfarbe aufweist, die der Farbe eines idealen kolorimetrischen Referenzindikators entspricht, wenn sie einer vorbestimmten CO2-Konzentration ausgesetzt ist, die nicht null ist, wobei, wenn das Kalibrierelement vor der Detektionseinheit (2) angeordnet ist, die Verarbeitungseinheit (12) ausgestaltet ist, die Lichtparameter derart anzupassen, dass die Kamera (6) die festgelegte Kalibrierungsfarbe detektiert, und die Steuersignalwerte, die erforderlich sind, um zu erreichen, dass die festgelegte Kalibrierungsfarbe detektiert wird, zu ermitteln und als kalibrierte Steuersignalwerte zu speichern, wobei die Verarbeitungseinheit (12) ausgestaltet ist, eine Kalibrierungsprozedur anzuwenden, die Folgendes umfasst:

- Anordnen eines zu verwendenden kolorimetrischen Indikators (10) in der Indikatorhalterung (8),
- Messen der Farbe der Oberfläche des kolorimetrischen Indikators, wenn diese von ausgestrahltem Licht beleuchtet wird, das unter Verwendung der ermittelten kalibrierten Steuersignalwerte erzeugt wird, wenn kein CO2 vorhanden ist, und Speichern der gemessenen Farbe als einen Null-CO2-Farbwert, und
- Ermitteln einer Beziehung von CO2-Konzentrationen basierend auf dem gemessenen Null-CO2-Farbwert, wobei die Verarbeitungseinheit (12) ausgestaltet ist, die Beziehung von CO2-Konzentrationen während Messungen von CO2-Konzentrationen anzuwenden, sofern die ermittelten kalibrierten Steuersignalwerte beim Ausstrahlen von Licht angewendet werden.

2. Kapnoskopanordnung nach Anspruch 1, wobei die Beleuchtungsanordnung (4) Leuchtdioden (LEDs) umfasst, die nahe der Kamera (6) angeordnet sind, vorzugsweise symmetrisch und/oder in Umfangsrichtung um die Kamera angeordnet, und wobei die Anordnung außerdem einen Beleuchtungsanpassungsadapter (18) umfasst, der ausgestaltet ist, gesteuert zu werden, um das ausgestrahlte Licht anzupassen, indem er Streulicht bereitstellt und eine Öffnung zur Begrenzung des Sichtfelds bereitstellt, um Bilder mit ausreichend homogenen Farbfeldern zu erhalten.

3. Kapnoskopanordnung nach Anspruch 1 oder 2, wobei die Indikatorhalterung (8) so ausgestaltet ist, dass sie, vorzugsweise durch ein Doppelklebeelement (20), lösbar an der Detektionseinheit (2) anbringbar und leicht davon abtrennbar ist, und mit einem Gaskanal (22) versehen ist, der so ausgestaltet ist, dass er es ermöglicht, dass ein zu messender Gasstrom die Indikatoroberfläche erreicht.

4. Kapnoskopanordnung nach einem beliebigen der Ansprüche 1-3, wobei die vorbestimmte CO2-Konzentration, die nicht null ist, innerhalb des Bereichs von 4-7 %, und vorzugsweise bei ungefähr 6 %, liegt.

5. Kapnoskopanordnung nach einem beliebigen der Ansprüche 1-4, wobei die festgelegte Kalibrierungsfarbe gemäß dem RGB-Farbcodesystem bestimmt ist, und dass nur einer der RGB-Parameter, vorzugsweise der R-Parameter, verwendet wird, um die Kalibrierungsfarbe zu bestimmen.

6. Kapnoskopanordnung nach einem beliebigen der Ansprüche 1-5, wobei die Beziehung der $CO_2$-Konzentrationen von R und der Differenz (Delta) zwischen dem gemessenen Null-$CO_2$-Farbwert und dem Null-$CO_2$-Farbwert des idealen kolorimetrischen Referenzindikators abhängig ist, wobei die $CO_2$-Konzentration anhand der Farbe des kolorimetrischen Indikators unter Verwendung der folgenden Formel berechnet wird: $C (CO_2) = A \times \ln (B / (B - (R - R0))$, wobei $C (CO_2)$ die $CO_2$-Konzentration ist, A und B Konstanten sind und R0 der R-Wert, wenn kein $CO_2$ vorhanden ist, ist, sofern ausschließlich der R-Parameter in dem RGB-Farbsystem verwendet wird.

7. Kapnoskopanordnung nach Anspruch 6, wobei die Beziehung der Konzentrationen eine lineare Form vom Typ R = R gemessen + Delta × (K1 + K2 × R gemessen) aufweist, wobei K1 und K2 Konstanten sind, und wobei die lineare Form für den R-Wert beim Berechnen der $CO_2$-Konzentration unter Verwendung der Formel in Anspruch 6 angewendet wird.

8. Verfahren in Verbindung mit einer Kapnoskopanordnung zur Messung einer Kohlendioxid($CO_2$)-Konzentration, wobei die Kapnoskopanordnung Folgendes umfasst:

   - eine Detektionseinheit (2), umfassend eine Beleuchtungsanordnung (4) und eine Kamera (6), die ausgestaltet ist, Farben einer Indikatoroberfläche zu erfassen, die mittels Licht beleuchtet wird, das von der Beleuchtungsanordnung ausgestrahlt wird, wobei die Beleuchtungsanordnung und die Kamera nah beieinander montiert sind,
   - eine Indikatorhalterung (8), die ausgestaltet ist, einen kolorimetrischen Indikator (10), der eine Indikatoroberfläche aufweist, vor der Detektionseinheit (2) in einer festgelegten Position in Bezug auf die Detektionseinheit (2) zu halten,
   - eine Verarbeitungseinheit (12), die ausgestaltet ist, Lichtparameter, z. B. die Farbe und Intensität, des von der Beleuchtungsanordnung (4) ausgestrahlten Lichts zu steuern, indem sie Steuersignale (14) erzeugt, **dadurch gekennzeichnet, dass** das Verfahren ein Aufnehmen und ein Halten eines Kalibrierelements (16), in der Indikatorhalterung (8), vor der Detektionseinheit (2) und in der festgelegten Position in Bezug auf die Detektionseinheit (2) umfasst, wobei das Kalibrierelement (16) eine Kalibrieroberfläche umfasst, die eine festgelegte Kalibrierungsfarbe aufweist, die der Farbe eines idealen kolorimetrischen Referenzindikators entspricht, wenn sie einer vorbestimmten $CO_2$-Konzentration ausgesetzt ist, die nicht null ist, wobei, wenn das Kalibrierelement vor der Detektionseinheit (2) angeordnet ist, das Verfahren ein Anpassen der Lichtparameter derart, dass die Kamera (6) die festgelegte Kalibrierungsfarbe detektiert, und ein Ermitteln und ein Speichern der Steuersignalwerte, die erforderlich sind, um zu erreichen, dass die festgelegte Kalibrierungsfarbe detektiert wird, als kalibrierte Steuersignalwerte und ein Anwenden einer Kalibrierungsprozedur umfasst, die Folgendes umfasst:

      - Anordnen eines zu verwendenden kolorimetrischen Indikators (10) in der Indikatorhalterung (8),
      - Messen der Farbe der Oberfläche des kolorimetrischen Indikators, wenn diese von ausgestrahltem Licht beleuchtet wird, das unter Verwendung der ermittelten kalibrierten Steuersignalwerte erzeugt wird, wenn kein $CO_2$ vorhanden ist, und Speichern der gemessenen Farbe als einen Null-$CO_2$-Farbwert, und
      - Ermitteln einer Beziehung der $CO_2$-Konzentrationen basierend auf dem gemessenen Null-$CO_2$-Farbwert, und wobei das Verfahren ferner ein Anwenden der Beziehung der $CO_2$-Konzentrationen während Mes-sungen von $CO_2$-Konzentrationen umfasst, sofern die ermittelten kalibrierten Steuersignalwerte beim Ausstrahlen von Licht angewendet werden.

9. Verfahren nach Anspruch 8, wobei die Beziehung der $CO_2$-Konzentrationen von R und der Differenz (Delta) zwischen dem gemessenen Null-$CO_2$-Farbwert und dem Null-$CO_2$-Farbwert des idealen kolorimetrischen Referenzindikators abhängig ist, wobei die $CO_2$-Farbkonzentration anhand der Farbe des kolorimetrischen Indikators unter Verwendung der folgenden Formel berechnet wird: $C (CO_2) = A \times \ln (B / (B - (R - R0))$, wobei $C (CO_2)$ die $CO_2$-Konzentration ist, A und B Konstanten sind und R0 der R-Wert, wenn kein $CO_2$ vorhanden ist, ist, sofern ausschließlich der R-Parameter in dem RGB-Farbsystem verwendet wird.

10. Verfahren nach Anspruch 9, wobei die Beziehung der Konzentrationen eine lineare Form vom Typ R = R gemessen + Delta × (K1 + K2 × R gemessen) aufweist, wobei K1 und K2 Konstanten sind, und wobei die lineare Form für den R-Wert beim Berechnen der $CO_2$-Konzentration unter Verwendung der Formel in Anspruch 9 angewendet wird.

**Revendications**

1. Ensemble capnoscope de mesure d'une concentration de dioxyde de carbone (CO2), comprenant :

   - une unité (2) de détection comprenant un agencement (4) d'éclairage et une caméra (6) configurée pour acquérir des couleurs d'une surface d'un indicateur éclairée par de la lumière émise par l'agencement d'éclairage, dans lequel l'agencement d'éclairage et la caméra sont montés ensemble en étant proches,
   - un support (8) de l'indicateur configuré pour supporter un indicateur (10) colorimétrique ayant une surface d'indicateur devant l'unité (2) de détection dans une position déterminée à l'avance par rapport à l'unité (2) de détection,
   - une unité (12) de traitement configurée pour commander des paramètres de lumière, par exemple la couleur et l'intensité, de la lumière émise par l'agencement (4) d'éclairage en produisant des signaux (14) de commande, **caractérisé en ce que** l'ensemble capnoscope comprend en outre un élément (16) d'étalonnage et **en ce que** le support (8) de l'indicateur est configuré pour recevoir et supporter l'élément (16) d'étalonnage devant l'unité (2) de détection et dans ladite position déterminée à l'avance par rapport à l'unité (2) de détection, l'élément (16) d'étalonnage comprend une surface d'étalonnage ayant une couleur d'étalonnage déterminée à l'avance correspondant à la couleur d'un indicateur colorimétrique idéal de référence, lorsqu'il est soumis à une concentration de CO2 définie à l'avance, qui n'est pas zéro, dans lequel l'élément d'étalonnage est disposé devant l'unité (2) de détection, l'unité (12) de traitement est configurée pour régler les paramètres de lumière, de manière à ce que la caméra (6) détecte la couleur d'étalonnage déterminée à l'avance, et pour déterminer et mettre en mémoire les valeurs de signal de commande requises pour obtenir que la couleur d'étalonnage déterminée à l'avance soit détectée, comme valeurs étalonnées de signal de commande, dans lequel l'unité (12) de traitement est configurée pour appliquer une procédure d'étalonnage, qui comprend :

     - disposer un indicateur (10) colorimétrique à utiliser dans le support (8) de l'indicateur,
     - mesurer la couleur de la surface de l'indicateur colorimétrique, lorsqu'elle est éclairée par de la lumière émise produite en utilisant les valeurs étalonnées déterminées de signal de commande, lorsque du CO2 n'est pas présent et mettre en mémoire la couleur mesurée, comme valeur zéro de couleur de CO2, et
     - déterminer une relation de concentration de CO2 sur la base de la valeur zéro mesurée de couleur de CO2, dans lequel l'unité (12) de traitement est configurée pour appliquer la relation de concentration de CO2 pendant des mesures de concentration de CO2, pourvu que les valeurs étalonnées déterminées de signal de commande soient appliquées lorsque de la lumière est émise.

2. Ensemble capnoscope suivant la revendication 1, dans lequel l'agencement (4) d'éclairage comprend des diodes électroluminescentes (LED) disposées près de la caméra (6), de préférence symétriquement et/ou circonférentiellement autour de la caméra, et dans lequel l'ensemble comprend aussi un adaptateur (18) de réglage de l'éclairage configuré pour être commandé pour régler la lumière émise, en donnant de la lumière diffusée et en donnant une ouverture pour limiter le champ de vue, afin d'obtenir des images de champ de couleur suffisamment homogènes.

3. Ensemble capnoscope suivant la revendication 1 ou 2, dans lequel le support (8) de l'indicateur est configuré pour être fixé, de manière amovible, à l'unité (2) de détection et en être détaché facilement, de préférence par un élément (20) adhésif double, et est pourvu d'un passage (22) pour du gaz configuré pour permettre à un courant de gaz à mesurer d'atteindre la surface de l'indicateur.

4. Ensemble capnoscope suivant l'une quelconque des revendications 1 à 3, dans lequel la concentration définie à l'avance de CO2, qui n'est pas zéro, est dans la plage de 4 à 7 %, et de préférence d'environ 6 %.

5. Ensemble capnoscope suivant l'une quelconque des revendications 1 à 4, dans lequel la couleur d'étalonnage déterminée à l'avance est définie suivant le système de code de couleur RGB et en ce que seulement l'un des paramètres RGB est utilisé pour définir la couleur d'étalonnage, de préférence le paramètre R.

6. Ensemble capnoscope suivant l'une quelconque des revendications 1 à 5, dans lequel la relation de concentration de CO2 dépend de R et de la différence (delta) entre la valeur zéro mesurée de couleur de CO2 et la valeur zéro de couleur de CO2 de l'indicateur colorimétrique idéal de référence, la concentration de CO2 est calculée à partir de la couleur de l'indicateur colorimétrique en utilisant la formule : $C(CO2) = A \times \ln(B/(B-(R-R0))$, dans laquelle $C(CO2)$ est la concentration de CO2, A et B sont des constantes et R0 est la valeur R quand du CO2 n'est pas présent, pourvu que seulement le paramètre R du système de couleur RGB soit utilisé.

**7.** Ensemble capnoscope suivant la revendication 6, dans lequel la relation de concentration a une forme linéaire du type R=R mesuré + delta × (K1 + K2 × R mesuré), K1 et K2 étant des constantes, et dans lequel la forme linéaire pour la valeur R est appliquée lorsque l'on calcule la concentration de CO2 en utilisant la formule de la revendication 6.

**8.** Procédé en liaison avec un ensemble capnoscope pour mesurer une concentration de dioxyde de carbone (CO2), l'ensemble capnoscope comprenant :

- une unité (2) de détection comprenant un agencement (4) d'éclairage et une caméra (6) configurée pour acquérir des couleurs d'une surface d'un indicateur éclairée par de la lumière émise par l'agencement d'éclairage, dans lequel l'agencement d'éclairage et la caméra sont montés ensemble en étant proches,
- un support (8) de l'indicateur configuré pour supporter un indicateur (10) colorimétrique ayant une surface d'indicateur devant l'unité (2) de détection dans une position déterminée à l'avance par rapport à l'unité (2) de détection,
- une unité (12) de traitement configurée pour commander des paramètres de lumière, par exemple la couleur et l'intensité, de la lumière émise par l'agencement (4) d'éclairage en produisant des signaux (14) de commande, **caractérisé en ce que** le procédé comprend en outre recevoir et maintenir un élément (16) d'étalonnage dans le support (8) de l'indicateur devant l'unité (2) de détection et dans ladite position déterminée à l'avance par rapport à l'unité (2) de détection, l'élément (16) d'étalonnage comprend une surface d'étalonnage ayant une couleur d'étalonnage déterminée à l'avance correspondant à la couleur d'un indicateur colorimétrique idéal de référence, lorsqu'il est soumis à une concentration de CO2 définie à l'avance, qui n'est pas zéro, dans lequel l'élément d'étalonnage est disposé devant l'unité (2) de détection, dans lequel le procédé comprend régler les paramètres de lumière, de manière à ce que la caméra (6) détecte la couleur d'étalonnage déterminée à l'avance, et déterminer et mettre en mémoire les valeurs de signal de commande requises pour obtenir que la couleur d'étalonnage déterminée à l'avance soit détectée, comme valeurs étalonnées de signal de commande, et appliquer une procédure d'étalonnage, qui comprend :

- mettre un indicateur (10) colorimétrique à utiliser dans le support (8) de l'indicateur,
- mesurer la couleur de la surface de l'indicateur colorimétrique, lorsqu'elle est éclairée par de la lumière émise produite en utilisant les valeurs étalonnées déterminées de signal de commande, lorsque du CO2 n'est pas présent, et mettre en mémoire la couleur mesurée, comme valeur zéro de couleur de CO2, et
- déterminer une relation de concentration de CO2 sur la base de la valeur zéro mesurée de couleur de CO2, le procédé comprend en outre appliquer la relation de concentration de CO2 pendant des mesures de concentration de CO2, pourvu que les valeurs étalonnées déterminées de signal de commande soient appliquées lorsque de la lumière est émise.

**9.** Procédé suivant la revendication 8, dans lequel la relation de concentration de CO2 dépend de R et de la différence (delta) entre la valeur zéro de couleur mesurée de CO2 et la valeur zéro de couleur de CO2 de l'indicateur colorimétrique idéal de référence, la concentration de CO2 est calculée à partir de la couleur de l'indicateur colorimétrique en utilisant la formule : C (CO2) = A × ln (B/(B-(R-R0)), dans laquelle C (CO2) est la concentration de CO2, A et B sont des constantes et R0 est la valeur R quand du CO2 n'est pas présent, pourvu que seulement le paramètre R du système de couleur RGB soit utilisé.

**10.** Procédé suivant la revendication 9, dans lequel la relation de concentration a une forme linéaire du type R=R mesuré + delta × (K1 + K2 × R mesuré), K1 et K2 étant des constantes, et dans lequel la forme linéaire pour la valeur R est appliquée lorsque l'on calcule la concentration de CO2 en utilisant la formule de la revendication 9.

FIG. 1

FIG. 2

FIG. 3

ARRANGING CALIBRATION MEMBER IN DETECTION UNIT, THE CALIBRATION MEMBER HAS CALIBRATION SURFACE WITH COLOUR OF IDEAL COLORIMETRIC INDICATOR SUBJECTED TO PREDEFINED NON-ZERO CO2 CONCENTRATION

↓

ADJUSTING LIGHT PARAMETERS SUCH THAT CAMERA DETECTS PREDETERMINED CALIBRATION COLOUR, DETERMINING AND STORING CALIBRATED CONTROL SIGNAL VALUES

↓

ARRANGING COLORIMETRIC INDICATOR IN DETECTION UNIT, MEASURING COLOUR WHEN ILLUMINATED BY LIGHT WHEN USING CALIBRATED CONTROL SIGNAL VALUES, AND NO CO2 IS PRESENT

↓

DETERMINING CO2 CONCENTRATION RELATIONSHIP BASED UPON MEASURED ZERO CO2 COLOUR VALUE

↓

APPLYING CO2 CONCENTRATION RELATIONSHIP DURING CO2 CONCENTRATION MEASUREMENTS PROVIDED THAT CALIBRATED CONTROL SIGNAL VALUES ARE USED

FIG. 4

**EP 3 982 827 B1**

REFERENCES CITED IN THE DESCRIPTION

**Patent documents cited in the description**

- US 10175254 B
- US 10080510 B
- US 20160331271 A